# EUROPEAN PATENT APPLICATION

(11) **EP 3 360 542 A1**
(43) Date of publication of application: **15.08.2018**
(21) Application number: 18156312.3
(22) Date of filing: 12.02.2018
(51) Int. Cl.: A61K 9/20, A61K 31/496

(54) **TABLET FORMS OF VILAZODONE HYDROCHLORIDE**

(30) Priority: 13.02.2017 TR 201702103
(71) Applicant: Sanovel Ilac Sanayi ve Ticaret A.S., 34460 Istanbul (TR)
(72) Inventor: TÜRKYILMAZ, Ali, 34460 Istanbul (TR); YILDIRIM, Ediz, 34460 Istanbul (TR); KÖKSAL UZUN, Selin, 34460 Istanbul (TR)
(74) Representative: Sevinç, Erkan

(57) **Abstract**

The present invention relates to a pharmaceutical tablet comprising vilazodone hydrochloride and at least one pharmaceutically acceptable excipient.

## Description

### Technical Aspect

The present invention relates to a pharmaceutical tablet comprising vilazodone hydrochloride and at least one pharmaceutically acceptable excipient.

### Background of the Invention

Vilazodone is used to treat depression. It is an SSRI (selective serotonin reuptake inhibitor) and partial serotonin receptor agonist. It works by helping to restore the balance of certain natural substances in the brain (neurotransmitters such as serotonin). This medication may improve your mood, sleep, appetite, and energy level and may help restore your interest in daily living.

The chemical designation of vilazodone is I-[4-(5-cyanoindol-3-yl) butyl]-4-(2-carbamoyl-benzofuran-5-yl)-piperazine hydrochloride. Its chemical structure is shown in the Formula I.

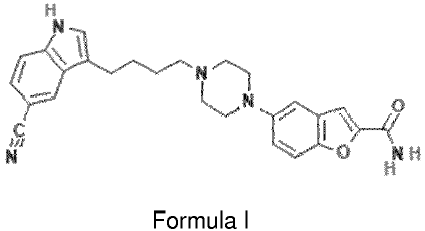

Vilazodone (commercially known as Viibryd®) is presently marketed as immediate-release tablets. Current guidelines for use of vilazodone in treatment of major depressive disorder recommends that vilazodone be administered at a starting dose of 10 mg once daily for seven days, followed by 20 mg once daily for additional seven days and then increased to 40 mg once daily.

Vilazodone hydrochloride is first disclosed in U.S. Patent No. 5,532,241.

Prior art shows that U.S. Patent No. 7,834,020 discloses fifteen crystalline modifications of vilazodone hydrochloride designated as Form I through Form XV. It also discloses conventional forms of administration such as tablets and capsules. PCT Publication No. WO 2012/131706 discloses the amorphous form of vilazodone hydrochloride. Further, PCT Publication No. WO 2013/078361 discloses several other polymorphic forms of vilazodone hydrochloride.

There still remains a need in the prior art to provide an improved pharmaceutical composition of viladozone hydrochloride, having a high solubility, dissolution rate, and therefore a high bioavailability and a long-term stability.

At present invention, the tablet comprising vilazodone hydrochloride has enough hardness to provide high chemical and mechanical stability, because it is not brittle easily.

### Description of the Invention

The main object of the present invention is to provide a formulation with high stability, having desired level of dissolution rate and compressibility which overcomes the above described problems in prior art and have additive advantages over them.

A further object of the present invention is to stabilize the active pharmaceutical ingredient which does not degrade to unwanted impurities.

Another object of the present invention is to provide high bioavailability and dissolution by the help of selection of excipients in a certain ratio.

Another object of the present invention is to provide a vilazodone hydrochloride tablet comprising at least one film coating to protect the pharmaceutical tablet against the moisture to maintain the stability.

According to this embodiment, the tablet comprises more than 21 mg vilazodone hydrochloride which has a hardness of more than 140 N, 11 mg- 20 mg vilazodone hydrochloride which has a hardness of more than 120 N, 1 mg - 10 mg vilazodone hydrochloride which has a hardness of more than 90 N.

According to this embodiment, the tablet comprises 21 mg - 40 mg, 41 mg - 60 mg, 61 mg- 80 mg, 81 mg - 90 mg vilazodone hydrochloride which has a hardness of more than 140 N.

According to this embodiment the tablet comprising more than 21 mg vilazodone hydrochloride has a hardness of more than 140 N, preferably between 145 N to 165 N, between 165 N to 185N, between 185 N to 205 N, between 205 N to 225 N, between 225 N to 250 N, between 250 N to 300 N. This provides high chemical and mechanical stability, thus it is not brittle easily.

According to this embodiment the tablet comprising 11 mg - 20 mg vilazodone hydrochloride has a hardness of more than 120 N, preferably between 125 N to 145 N, between 145 N to 165N, between 165 N to 185 N, between 185 N to 205 N, between 205 N to 225 N, between 225 N to 250 N, between 250 N to 280 N. This provides high chemical and mechanical stability, thus it is not brittle easily.

According to this embodiment the tablet comprising 1 mg -10 mg vilazodone hydrochloride has a hardness of more than 90 N, preferably between 95 N to 115 N, between 115 N to 135N, between 135 N to 155 N, between 155 N to 175 N, between 175 N to 195 N, between 195 N to 220 N, between 220 N to 250 N. This provides high chemical and mechanical stability, thus it is not brittle easily.

According to another embodiment, the total amount of vilazodone hydrochloride in the tablet is 2.0% to 45.0% by weight. Preferably it is 6.0% to 18.0% by weight.

At present invention, vilazodone hydrochloride has a d (0.5) particle size not less than 20 µm, d (0.1) particle size not less than 6 µm, d (0.9) particle size not less than 30 µm.

In general terms, excipients provided in a formulation may positively or negatively influence the physicochemical and pharmacokinetic properties, e.g. the solubility, absorption, bioavailability of an active agent. For this reason, the excipients which accompany an active agent have to be selected in a careful and conscious manner while a formulation is developed. The formulations should have no physicochemical incompatibility between the drug and the excipients.

According to this embodiment of the present invention, the tablet further comprises at least one pharmaceutically acceptable excipient selected from disintegrants, fillers, binders, lubricants, glidants or mixtures thereof.

Suitable disintegrants are selected from group comprising crospovidone, ion exchange resin, hydroxypropylcellulose, croscarmellose sodium, starches, pectins, alginates, magnesium silicate, aluminum silicate, microcrystalline cellulose, sodium starch glycolate or mixtures thereof, preferably the disintegrant is crospovidone. The amount of the crospovidone in the tablet is 2.0% w/w to 10.5% w/w by weight.

The ratio of crospovidone to the other excipients are very important for this invention to improve the quality of the film-coated tablet, since it is known that disintegrants have tablet quality deteriorating properties, such as causing tablet hardness reductions and tablet surface roughness as a result of moisture absorption, and worsening the mouth touch due to a feeling of dryness as a result of saliva absorption. Regarding this knowledge, in this invention, crospovidone is used in minimal ratios as given above as the single disintegrant.

Suitable fillers or binders are selected from the group comprising lactose monohydrate, microcrystalline cellulose, lactose anhydrous, pregelatinized starch, calcium carbonate, calcium phosphate dibasic, calcium phosphate tribasic, calcium sulphate, kaolin, starch or mixtures thereof, preferably fillers or binders are lactose monohydrate and microcrystalline cellulose. The total amount of the fillers or binders in the tablet 10.0% w/w to 90.0% w/w by weight

According to this embodiment of the present invention, the total amount of microcrystalline cellulose in the tablet is 5.0% to 40.0% by weight.

According to this embodiment of the present invention, crospovidone is very effective excipient in the preparation of the tablet. It has been surprisingly found that the object of the present invention is achieved when crospovidone and microcrystalline cellulose are used in a certain ratio. Consequently, the ratio of microcrystalline cellulose to crospovidone may be in the range of 0.01-10.0 by weight, preferably the ratio is 1.0-6.0. Said ratio provides adequate disintegrating properties for the tablets of the present invention but also stabilizes the active pharmaceutical ingredient which does not degrade to unwanted impurities.

Suitable lubricants are selected from group comprising magnesium stearate, stearic acid, calcium stearate, zinc stearate, sodium stearyl fumarate, hydrogenated castor oil, hydrogenated vegetable oil, light mineral oil, mineral oil, polyethylene glycol, sodium benzoate or mixtures thereof, preferably lubricant is magnesium stearate. The total amount of the lubricants in the tablet is 0.01% w/w to 10.0% w/w by weight.

Suitable glidants are selected from group comprising colloidal silicon dioxide, water soluble excipient, hydrophilic polymers, silicon dioxide or mixtures thereof, preferably glidant is colloidal silicon dioxide. The total amount of the glidant in the tablet is 0.01% w/w to 10.0% w/w by weight.

In one embodiment of the present invention, the pharmaceutical tablet is in the form of coated tablet, film- coated tablet, trilayer tablet, bilayer tablet, multilayer tablet, orally disintegrating tablet, mini tablet, pellet, buccal tablet, sublingual tablet, effervescent tablet, immediate release tablet, modified release tablet. Preferably, the pharmaceutical tablet composition is in the form of film- coated tablet.

According to this embodiment, the pharmaceutical tablet is in the form of a film-coated tablet which comprises polyvinyl alcohol, titanium dioxide, polyethylene glycol, talc, coloring agent or mixtures thereof.

Suitable coloring agents are selected from group comprising FD&C Blue, FD&C Yellow, FD&C Red, Quinoline yellow aluminium lake or mixtures thereof.

In one embodiment of the present invention, the tablet comprises 1.0-70.0% by weight of vilazodone hydrochloride, 25-80.0% by weight of lactose monohydrate, 5.0-40.0% by weight of microcrystalline cellulose, 2.0-10.5% by weight of crospovidone, 0.01-10.0% by weight of magnesium stearate, 0.01-10.0% by weight of colloidal silicon dioxide, 1.0 - 5.0% by weight of coating.

In one embodiment of the present invention, the pharmaceutical tablet comprising vilazodone hydrochloride is prepared by direct compression method.

According to this embodiment, a process for preparing the pharmaceutical tablet comprises the following steps:
a) sieving vilazodone hydrochloride, lactose monohydrate, microcrystalline cellulose, crospovidone and colloidal silicon dioxide and mixing them for 15 minutes
b) adding magnesium stearate and mixing the total mixture for 5 minutes
c) compressing the total mixture into tablets
d) coating with film coating the tablet, which comprising polyvinyl alcohol, titanium dioxide, polyethylene glycol, talc, coloring agent.

### Example

This example describes the pharmaceutical tablet comprising vilazodone hydrochloride. The active ingredient and excipients of the tablet are given below:

### Example 1: The pharmaceutical tablet of vilazodone hydrochloride

| **Content** | Amount (% by weight of the total) |
|---|---|
| vilazodone hydrochloride | 6.0 - 18.0 |
| lactose monohydrate | 40.0 - 70.0 |
| microcrystalline cellulose | 10.0 - 30.0 |
| crospovidone | 3.0 - 10.0 |
| magnesium stearate | 0.1 - 8.0 |
| colloidal silicon dioxide | 0.05 - 8.0 |
| coating | 1.0 - 5.0 |

| **Coating Material Ingredients** | **Amount (% by weight of the coating)** |
|---|---|
| polyvinyl alcohol | 30.0 - 50.0 |
| titanium dioxide | 17.0 - 35.0 |
| talc | 8.0 - 25.0 |
| polyethylene glycol | 12.0 - 32.0 |
| coloring agent | 0.01 -5.0 |

### Process for example

A process for preparing the pharmaceutical tablet in example 1 comprises the following steps:
a) sieving vilazodone hydrochloride, lactose monohydrate (spray-dried 11SD), microcrystalline cellulose Ph 102, crospovidone CL and colloidal silicon dioxide and mixing them for 15 minute
b) adding magnesium stearate and mixing the total mixture for 5 minutes
c) compressing the total mixture into tablets
d) coating with film coating the tablet, which comprising polyvinyl alcohol, titanium dioxide, polyethylene glycol, talc, coloring agent.

## Claims

1. A pharmaceutical tablet comprising more than 21 mg vilazodone hydrochloride which has a hardness of more than 140 N, 11 mg- 20 mg vilazodone hydrochloride which has a hardness of more than 120 N, 1 mg - 10 mg vilazodone hydrochloride which has a hardness of more than 90 N.

2. The pharmaceutical tablet according to claim 1, wherein the tablet comprises 40 mg vilazodone hydrochloride which has a hardness of more than 140 N, 20 mg vilazodone hydrochloride which has a hardness of more than 120 N, 10 mg vilazodone hydrochloride which has a hardness of more than 90 N.

3. The pharmaceutical tablet according to claim 2, wherein the tablet comprises 40 mg vilazodone hydrochloride which has a hardness between 145 N to 300 N, 20 mg vilazodone hydrochloride which has a hardness between 120 N to 280 N, 10 mg vilazodone hydrochloride which has a hardness between 90 N to 250 N.

4. The pharmaceutical tablet according to claim 3, wherein the total amount of the vilazodone hydrochloride in the tablet is 2.0% to 45.0% by weight.

5. The pharmaceutical tablet according to claim 4, wherein the total amount of the vilazodone hydrochloride in the tablet is 6.0% to 18.0% by weight.

6. The pharmaceutical tablet according to claim 5, wherein the tablet comprisesvilazodone hydrochloride which has a d (0.5) particle size not less than 20 µm and d (0.1) particle size not less than 6 µm and d (0.9) particle size not less than 30 µm.

7. The pharmaceutical tablet according to any preceding claims, wherein the tablet further comprises at least one pharmaceutically acceptable excipient selected from disintegrans, binders, fillers, lubricants, glidants or mixtures thereof.

8. The pharmaceutical tablet according to the claim 7, wherein the disintegrants are selected from the group comprising crospovidone, ion exchange resin, hydroxypropylcellulose, croscarmellose sodium, starches, pectins, alginates, magnesium silicate, aluminum silicate, microcrystalline cellulose, sodium starch glycolate or mixtures thereof, preferably the disintegrant is crospovidone.

9. The pharmaceutical tablet according to the claim 8, wherein the total amount of the crospovidone in the tablet is 2.0% to 10.5% by weight.

10. The pharmaceutical tablet according to the claim 7, wherein fillers or binders are selected from the group comprising lactose monohydrate, microcrystalline cellulose, lactose anhydrous, pregelatinized starch, calcium carbonate, calcium phosphate dibasic, calcium phosphate tribasic, calcium sulphate, kaolin, starch or mixtures thereof, preferably fillers or binders are lactose monohydrate and microcrystalline cellulose.

11. The pharmaceutical tablet according to the claim 10, wherein the total amount of the microcrystalline cellulose in the tablet is 5.0% to 40% by weight.

12. The pharmaceutical tablet according to any preceding claims, wherein the weight ratio of microcrystalline cellulose to crospovidone is 0.01-10.0, preferably the ratio is 1.0-6.0.

13. The pharmaceutical tablet according to any preceding claims, wherein the tablet is in the form of coated tablet, film-coated tablet, trilayer tablet, bilayer tablet, multilayer tablet, orally disintegrating tablet, mini tablet, pellet, buccal tablet, sublingual tablet, effervescent tablet, immediate release tablet, modified release tablet.

14. The pharmaceutical tablet according to claim 13, wherein the tablet is in the form of a film-coated tablet.

15. The pharmaceutical tablet according to any preceding claims, wherein the tablet comprises;
1.0-70.0% by weight of vilazodone hydrochloride,
25-80.0% by weight of lactose monohydrate,
5.0-40.0% by weight of microcrystalline cellulose,
2.0-10.5% by weight of crospovidone,
0.01-10.0% by weight of magnesium stearate,
0.01-10.0% by weight of colloidal silicon dioxide
1.0 - 5.0% by weight of coating.

16. A process for preparing the pharmaceutical tablet according to claim 15, wherein the tablet comprises the following steps:
a) sieving vilazodone hydrochloride, lactose monohydrate, microcrystalline cellulose, crospovidone and colloidal silicon dioxide and mixing them for 15 minutes
b) adding magnesium stearate and mixing the total mixture for 5 minutes
c) compressing the total mixture into tablets
d) coating with film coating the tablet, which comprising polyvinyl alcohol, titanium dioxide, polyethylene glycol, talc, coloring agent.
